# EUROPEAN PATENT APPLICATION

(11) **EP 1 113 081 A1**
(43) Date of publication of application: **04.07.2001**
(21) Application number: 99403304.1
(22) Date of filing: 28.12.1999
(51) Int. Cl.: C12Q 1/68, C12N 5/00, C12N 15/00, A01K 67/027

(54) **Implication of a known gene named CP2/LSF/LBP-1 in Alzheimer's disease**

(71) Applicant: INSTITUT PASTEUR DE LILLE, 59019 Lille Cédex (FR); INSTITUT NATIONAL DE LA SANTE ET DE LA RECHERCHE MEDICALE (INSERM), 75013 Paris (FR)
(72) Inventor: Chartier-Harlin, Marie-Christine, 59139 Wattignies (FR); Amouyel, Philippe, 59700 Marcq-en-Baroeul (FR); Lambert, Jean-Charles, Dept. of Psychiatry, Brimingham B15 2QZ (GB)
(74) Representative: Hirsch, Denise

(57) **Abstract**

This invention concerns a method of predicting an increased risk of a human subject of developing Alzheimer's disease comprising assaying for a mutation within the ADN sequence of the CP2/LSF/LBP-1 gene including the region controlling the expression of said gene.

## Description

The invention relates to the implication of a known gene named CP2/LSF-LBP-1 in Alzheimer's disease, namely in sporadic forms of Alzheimer's disease.

Alzheimer's disease is a neurodegenerative process characterised by two main cerebral lesions : amyloid plaques and neurofibrillary degeneration. Numerous data suggested that the first step of the development of the disease would be the formation of the senile plaques, leading to neurodegeneration and finally to dementia. To better understand the etiology of the pathology, a systematic search for genes responsible of the monogenic familial forms of Alzheimer's disease have been undertaken. At present, three genes have been discovered, bearing pathogenic mutations : the APP gene on chromosome 21 (Goate et al, 1991), the PS1 and PS2 genes on chromosome 14 and 1 respectively (Sherrington et al, 1995 ; Levy-Lahad et al, 1995).

Even if the discovery of these mutations were essential for the understanding of the Alzheimer's disease process, they explain only less than 2% of all Alzheimer's disease cases (Cruts et al, 1998). Indeed, most of the forms are sporadic, without obvious mendelian inheritance. To date, only one gene has been recognized as a major risk factor for the sporadic form, the APOE gene on chromosome 19, the α4 allele of this gene strongly increasing the risk of developing the disease (Saunders et al, 1993). Furthermore, expression of this gene may be an important determinant for the disease, mutations in the APOE promoter modulating the disease risk (Bullido et al, 1998; Lambert et al, 1998).

More particularly, WO 99/01 574 discloses a method of predicting an increased risk of developing Alzheimer's disease consisting of identifying one or more mutations in the region of genomic ADN regulating the expression of APOE, the mutation(s) being responsible for a modified expression of APOE with respect to a standard population or a modified expression of one allele of APOE with respect to the other.

The mutation is namely a G→T replacement in the promoter region of the APOE gene located 186 bases upstream of the TATA box of said gene, in a potential binding site of the transcription factors Th1/E47CS.

However, APOE locus can only explain less than 50 % of the Alzheimer's disease cases (Farrer et al, 1997) and other genetic determinants should exist.

Recently, three reports described a novel locus on chromosome 12 (Pericak-Vance et al, 1997 ; Wu et al, 1998 ; Rogaeva et al, 1998). Two very plausible candidates genes have been studied in the region of interest: the α2-macroglobulin and the lipoprotein -receptor-related protein (LRP) (Blacker et al, 1998 ; Lendon et al, 1997). However, numerous data seem to exclude the first in sporadic cases, while the second could be only a minor contributor to the disease risk (Lambert et al, 1998 ; Wavrant-de Vrièze et al, 1999). So, the gene located on chromosome 12 and implicated in Alzheimer's disease, is still unknown.

According to the present invention, it has now been discovered that the gene located on chromosome 12 and implicated in Alzheimer's disease is the CP2/LSF/LBP-1 gene previously identified and characterized (Lim et al, 1992 ; Swendeman et al, 1994).

The CP2/LSF/LBP-1 gene comprises 15 exons and encodes a cellular transcription factor that interacts with the α-globulin promoter (Lim et al, 1992) as well as with additional cellular and viral promoter elements including the γ-fibrinogen promoter (Chodosh et al, 1988), the promoter of HIV-1 (Kato et al, 1991) and the SV-40 major late promoter (Huang et al, 1990).

Recently it has been reported to enhance the transcription of the serum amyloid A3 (SAA3) gene which encodes a major acute-phase protein synthesized and secreted manly by the liver (Bing et al, 1999).

It has further been reported that the CP2/LSF/LBP-1 factor interacted with the Fe65 protein, suspected to control the internalisation and the processing of the APP (Lambrano et al, 1998).

However, its implication with Alzheimer's disease at a genetic level has not been suggested prior to the instant invention.

The inventor's studies that have led to the instant invention show that mutations in the ADN sequence of the CP2/LSF/LBP-1 gene including the region controlling the expression of said gene, namely the promoter substantially increase the risk of a human subject of developing Alzheimer's disease.

The inventors have namely identified numerous silent polymorphisms, particularly polymorphisms located in untranslated regions of the CP2/LSF/LBP-1 gene.

The polymorphisms were namely located in the promoter, the 5' untranslated region of exon 1 in the 3' untranslated region of exon 15.

One of the polymorphisms located in exon 15 of the untranslated region revealed to be correlated with a substantial increased risk of developing Alzheimer's disease.

This polymorphism consists in a G→A mutation at position +15 of the stop codon of the CP2/LSF-LBP-1 gene.

According to a first aspect, the present invention provides a method of predicting an increased risk of a human subject of developing Alzheimer's disease comprising assaying for a mutation within the ADN sequence of the CP2/LSF/LBP-1 gene including the region controlling the expression of said gene.

In the sense of the instant invention, a mutation may consist of a simple base replacement, an insertion or deletion of one or more bases.

According to the instant invention, a mutation includes an allelic variant or polymorphic form of the CP2/LSF/LBP-1 gene locus, defined with reference to the most frequent allele in the general population.

When present in the coding sequence, the mutation may or may not result in an amino acid substitution, deletion or addition.

The mutation may be present in an non coding sequence of the gene. Thus, the mutation may be present in the regulatory sequences of the gene, namely in the promoter of the gene.

It may be located in a intron or in the 5' untranslated region of exon 1 or in the 3' untranslated region of exon 15 of the CP2/LSF/LBP-1 gene.

According to a preferred embodiment, the method of the invention consists of identifying the presence of a base other than G at position +15 of the stop codon of exon 15 of the CP2/LSF/LBP-1 gene, namely the presence of an Adenine residue in said position.

In order to detect the presence of a CP2/LSF/LBP-1 allele predisposing to Alzheimer's disease, a biological sample such as blood is prepared and analyzed for the presence or absence of susceptibility alleles of CP2/LSF/LBP-1 gene.

Initially the screening method involves amplification of the relevant CP2/LSF/LBP-1 sequences.

In another preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

The most popular method used today is target amplification. Here, the target nucleic acid sequence is amplified with polymerases. One particularly preferred method using polymerase-driven amplification is the polymerase chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

In one preferred embodiment, the method of the invention comprises the amplification of a region of interest suspected to comprise said mutation, by a polymerase chain reaction by means of oligonucleotide primers, wherein the reaction product comprises polynucleotides of at least 25 contigous nucleotides from said CP2/LSF-LBP-1 gene.

The method may further comprise sequencing gel reaction products from a PCR or RT-PCR reaction, namely the PCR *Bsp*12861digest from the PCR or RT-PCR reaction.

When the probes are used to detect the presence of the target sequences (for example, in screening for Alzheimer's disease susceptibility), the biological sample to be analyzed, such as blood, may be treated, if desired, to extract the nucleic acids. The sample nucleic acid may be prepared in various ways to facilitate detection of the target sequence ; e.g. denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 12. Therefore, high stringency conditions are generally desirable in order to prevent false positives.

The stringency of hybridization is determined by a number of factors during hybridization and during the washing procedure, including temperature, ionic strength, base composition, probe length, and concentration of formamide. These factors are outlined in, for example, Maniatis et al, 1982 and Sambrook et al, 1989. Under certain circumstances, the formation of higher order hybrids, such as triplexes, quadraplexes, etc may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels and methods for labeling probes and ligands are known in the art, and include, for example, radioactive labels which may be incorporated by known methods (e.g. nick translation, random priming or kinasing), biotin, fluorescent groups, chemiluminescent groups (e.g. dioxetanes, particularly triggered dioxetanes), enzymes, antibodies and the like. Variations of this basic scheme are known in the art, and include those variations that facilitate separation of the hybrids to be detected for extraneous materials and/or that amplify the signal from the labeled moiety. A number of these variations are reviewed in e.g. Matthews & Kricka, 1988 ; Landegren et al, 1988 ; Mittlin, 1989 ; US 4,868,105 and in EP 225,807.

As noted above, non-PCR based screening assays are also contemplated in this invention. One procedure hybridizes a nucleic acid probe (or an analog such as a methyl phosphonate backbone replacing the normal phosphodiester), to the low level DNA target. This probe may have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in a 103-106 increase in sensitivity. For an example relating to the preparation of oligodeoxynucleotide-alkaline phosphatase conjugates and their use as hybridization probes (see Jablonski et al, 1986).

Two-step label amplification methodologies are known in the art. These assays work on the principle that a small ligand (such as digoxigenin, biotin, or the like) is attached to a nucleic acid probe capable of specifically binding CP2/LSF-LBP-1. Allele specific probes are also contemplated within the scope of this example and exemplary allele specific probes include probes encompassing the predisposing mutation.

In one example, the small ligand attached to the nucleic acid probe is specifically recognized by an antibody-enzyme conjugate.

In one embodiment of this example digoxigenin is attached to the nucleic acid probe. Hybridization is detected by an antibody-alkaline phosphatase conjugate which turns over a chemiluminescent substrate. For methods for labeling nucleic acid probes according to this embodiment (see Martin et al, 1990).

In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays (see Rigby et al, 1977 and Nguyen et al, 1992).

It is also contemplated within the scope of this invention that the nucleic acid probe assays of this invention will employ a cocktail of nucleic acid probes capable of detecting CP2/LSF-LBP-1 gene. Thus, in one example to detect the presence of CP2/LSF-LBP-1 gene in a cell sample, more than one probe complementary to CP2/LSF-LBP-1 is employed and in particular the number of different probes in alternatively 2, 3 or 5 different nucleic acid probe sequences. In another example, to detect the presence of mutations in the CP2/LSF-LBP-1 gene sequence in a patient more than one probe complementary to CP2/LSF-LBP-1 is employed where the cocktail includes probes capable of binding to the allele-specific mutations identified in populations of patients with alteration in CP2/LSF-LBP-1. In this embodiment, any number of probes can be used, and will preferably include probes corresponding to the major gene mutations identified as predisposing an individual to Alzheimer's disease. Some candidate probes include probes that include allele-specific mutations.

The determination of the alleles of the newly-identified polymorphisms may alternatively be performed by creating in one of the primers used for the PCR method one cleavage site for a restriction enzyme which does not exist in the ADN of subjects bearing one of the alleles.

The method of the instant invention may also comprise one or more of the following steps :
- determining the genotype of the apolipoprotein E, the presence of at least one copy of the ε4 allele being indicative of an increased risk of developing Alzheimer's disease ;
- determining the genotype of the Th1/E47cs polymorphism, the presence of at least one copy of the G allele (T→G mutation in position -186 from the TATA box of the APOE gene) being indicative of an increased risk of developing Alzheimer's disease.

The genotype of the apolipoprotein E may be determined as disclosed in WO 95/29 257.

The genotype of the Th1/E47cs polymorphism may be determined as disclosed in WO 99/01 574.

According to a further aspect, the instant invention encompasses a method of screening for an agent capable of treating Alzheimer's disease comprising contacting a cultured cell line comprising a mutation or an allelic variant of the CP2/LSF-LBP-1 gene with an agent capable of treating Alzheimer's disease and monitoring the expression or processing of proteins encoded by the mutated form or allelic variant of the CP2/LSF-LBP-1 gene.

The cell line comprising a mutation or allelic variant of the CP2/LSF-LBP-1 gene advantageously consists of a transgenic host.

The transgenic host may be an immortalized eucaryotic cell line such as Neuroblastoma cell lines Kelly.

Alternatively, the transgenic host may be a bacterium.

Preferably, the host is obtained by integrating by means of known procedures a nucleic acid segment encompassing the mutated region of the CP2/LSF-LBP-1 gene or cDNA comprising a mutated region of the CP2/LSF-LBP-1 gene into the host genome.

According to a further aspect, the invention provides isolated nucleic acids comprising at least 15, preferably at least 20 consecutive nucleotides of the cDNA sequence of the LBP-1 gene having at least one of the following mutations or polymorphisms;
G (+ 637) → A (exon 1)
G (+ 2089) → A (exon 15)

The cDNA sequence is available on EMBL under access number M84810.

A preferred isolated nucleic acid according to the invention comprises at least 15, preferably at least 20 consecutive nucleotides having the G (+ 2089) →A polymorphism.

The invention further provides isolated nucleic acids selected from:
- nucleic acids comprising at least 15, preferably at least 20 consecutive nucleotides of the promoter sequence of the LBP-1 gene comprising the G (+120) → A polymorphism. The sequence is available on EMBL under access number U01965.
- nucleic acids comprising at least 15, preferably at least 20 consecutive nucleotides of sequence SEQ ID n° 1 (intron 2) comprising one or more polymorphisms selected from A (+ 174) → G and G (+ 660) → C ;
- nucleic acids comprising at least 15, preferably at least 20 consecutive nucleotides of sequence SEQ ID n° 2 (intron 3) comprising one or more polymorphisms selected from T (+ 210) → C and T (+ 431) → G;
- nucleic acids comprising at least 15, preferably at least 20 consecutive nucleotides of sequence SEQ ID n° 3 (intron 7) comprising the C (+134) → A polymorphism ;
- nucleic acids comprising at least 15, preferably at least 20 consecutive nucleotides of sequence SEQ ID n° 4 (intron 9) comprising the T (-27) → C polymorphism ;
- nucleic acids comprising at least 15, preferably at least 20 consecutive nucleotides of sequence SEQ ID n° 5 (intron 10) comprising the T (-14) → C polymorphism.

The nucleotide sequences SEQ ID n° 1-5 are represented in the enclosed sequence listing :

The nucleotides which are the subject matter of the polymorphisms mentioned above are numbered from the nucleotides in bold letters in SEQ ID n° 1 to 5, up to the right when the nucleotide is specified (+) and down to the left when the nucleotide is specified (-).

"Isolated nucleic acid" is one which is substantially separated from other cellular components which naturally accompany a native human sequence or protein (e.g, ribosomes, polymerases, many other human genome sequences and proteins). The term embraces a nucleic acid sequence which has been removed from its naturally occurring and includes recombinant or cloned DNA isolates and chemically synthesized analogs or analogs biologically synthesized by heterologous systems.

The polynucleotide compositions of this invention include RNA, cDNA, genomic DNA as well as synthetic forms, and mixed polymers and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g. methyl phosphonates, phosphodiesters, phosphoamidates, carbamates, etc) charged linkages (e.g. phosphorothioates, phosphorodithioates, etc), pendent moieties (e.g. polypeptides), intercalators (e.g. acridine, psoralen, etc), chelators, alkylators, and modified linkages (e.g., alpha anomeric nucleic acids, etc).

The present invention provides recombinant nucleic acids as hereindefined comprising all or part of the CP2/LSF-LBP-1 region. The recombinant construct may be capable of replicating autonormously in a host cell. Alternatively, the recombinant construct may become integrated into the chromosomal DNA of the host cell. Such a recombinant polynucleotide comprises a polynucleotide of genomic, DNA, semi-synthetic, or synthetic origin which, by virtue of its origin or manipulation, 1) is not associated with all or a portion of a polynucleotide with which it is associated in nature ; 2) is linked to a polynucleotide other than that to which it is linked in nature ; 3) does not occur in nature.

Therefore, recombinant nucleic acids comprising sequences otherwise not naturally occurring are provided by this invention.

According to a further aspect, the invention provides a vector comprising one of the nucleic acids specified hereabove.

The invention further provides host cells transformed wit such a vector.

Large amounts of the polynucleotides of the present invention may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually, the polynucleotide constructs will be suitable for replication in an unicellular host, such as yeast or bacteria, but may also be intended for introduction to (with and without integration within the genome) cultured mammalian or plant or other eukaryotic cell lines. The purification of nucleic acids produced by the methods of the present invention is described, e.g. in Sambrook et al, 1989 or Ausubel et al, 1992.

The polynucleotides of the present invention may also be produced by chemical synthesis, e.g. by the phosphoramidite method described by Beaucage & Carruthers, 1981 or the triester method according to Matteuci and Carruthers, 1981 and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

Polynucleotide constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals may also be included where appropriate, whether from a native CP2/LSF-LBP-1 protein or from other receptors or from secreted polypeptides of the same or related species, which allow the protein to cross and/or lodge in cell membranes, and thus attain its functional topology, or be secreted from the cell. Such vectors may be prepared by means of standard recombinant techniques well known in the art and discussed, for example, in Sambrook et al, 1989 or Ausubel et al, 1992.

An appropriate promoter and other necessary vector sequences will be selected so as to be functional in the host, and may include, when appropriate, those naturally associated with CP2/LSF-LBP-1 genes. Examples of workable combinations of cell lines and expression vectors are described in Sambrok et al, 1989 or Ausubel et al, 1992 ; see also, e.g. Metzger et al, 1988. Many useful vectors are known in the art and may be obtained from such vendors as Stratagene, New England Biolabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization an others. Vectors and promoters suitable for use in yeast expression are further described in Hitzeman et al, EP 73 675A. Appropriate non-native mammalian promoters might include the early and late promoters from SV40 (Fiefs et al, 1978) or promoters derived from murine Moloney leukemia virus, mouse tumor virus, avian sarcoma viruses, adenovirus II, bovine papilloma virus or polyoma. In addition, the construct may be joined to an amplifiable gene (e.g. DHFR) so that multiple copies of the gene may be made. For appropriate enhancer and other expression control sequences, see also *Enhancers and Eukaryotic* Gene *Expression,* Cold Spring Harbor Press, Cold Spring Harbor, N. Y. (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encodes proteins that a) confer resistance to antibiotics or other toxic substances, e.g. ampicillin, neomycin, methotrexate, etc ; b) complement auxotrophic deficiencies, or c) supply critical nutrients not available from complex media, e. g. the gene encoding D-alanine racemase for Bacilli. The choice of the proper selectable marker will depend on the host cell, and appropriate markers for different hosts are well known in the art.

The vectors containing the nucleic acids of interest can be transcribed *in vitro,* and the resulting RNA introduced into the host cell by well-known methods, e. g. by injection (Kubo et al, 1988), or the vectors can be introduced directly into host cells by methods well known in the art, which vary depending on the type of cellular host, including electroporation, transfection employing calcium chloride, rubidium chloride, calcium phosphate, DEAE-dextran, or other substances ; microprojectile bombardment ; lipofection ; infection (where the vector is an infectious agent, such as a retroviral genome); and other methods (generally, Sambrook et al, 1989 and Ausubel et al, 1992). The introduction of the polynucleotides into the host cell by any method known in the art, including, inter alia, those described above, will be referred to herein as "transformation". The cells into which have been introduced nucleic acids described above are meant to also include the progeny of such cells.

The invention also provides a transgenic non human animal, preferably mammal with germlines or somatic cells comprising a heterologous gene encompassing a mutated CP2/LSF-LBP-1 gene, namely the CP2/LSF-LBP-1 exon 15 mutant as defined above.

The host as well as the transgenic non human animals may be useful for screening for drugs capable of reducing or treating symptoms associated with the Alzheimer's disease in patients in need of such treatment.

Furthermore, it is well known that patients suffering from Alzheimer's disease show a pre-disposition to respond to certain therapies, namely to cholinomimetic-based therapies, depending on the type and number of copies of the APOE gene alleles (WO 95/29 257).

The instant invention further provides a method for the identification of human subjects suffering from Alzheimer's disease to be responsive to a given therapy, namely cholinomimetic therapy comprising :
a) determining the genotype of the CP2/LSF-LBP-1 gene.
   The method optionally further comprises one or more of the following steps :
b) determining the genotype of the apolipoprotein E ;
c) determining the genotype of the Th1/E47cs polymorphism ; and
d) determining the phase of the apolipoprotein E and Th1/E47cs polymorphism.

The following examples and figures are intended to illustrate the invention.

In the annexed figures,
- Figure 1 represents a comparison between sequences of the APOE promoter region encompassing the -216 GT polymorphism and an HIV regulatory element, containing a CP2/LSF-LBP-1 binding domains (Parado et al, 1995) ; similar consensus sequences were indicated in bold.
- Figure 2 (a) illustrates protein binding of cytoplasmic and nuclear extracts from different cell lines (N : neuroblastoma ; A : astrocytoma ; I : lymphocyte ; H : hepatoma). (b) amplification of CP2 and β-actin mRNA by RT-PCR in the different cell lines.
- Figure 3 (a) illustrates competition of nuclear protein binding by excess of cold oligonucleotides at equilibrium. (b) relative binding of each allele of the CP2 exon 15 polymorphism for an increase of cold oligonucleotides at the equilibrium.
- Figure 4 illustrates cytoplasmic or nuclear protein binding from lymphocytes treated without (-) or with (+) PHA.
- Figure 5(a) illustrates the mRNA expression pattern of the mRNA of the GG and GA phenotypes of exon 15 for lymphocytes of control and Alzheimer's disease cases.
- Figure 5(b) represents the ratio between the amount of the CP2 and β-actin mRNA for lymphocytes of control and Alzheimer's disease cases.

### Example 1 :

### Polymorphisms of CP2/LSF-LBP-1 gene

We sequenced the entire gene of CP2/LSF/LBP-1 using the oligonucleotides as described in Tables 1 and 2.

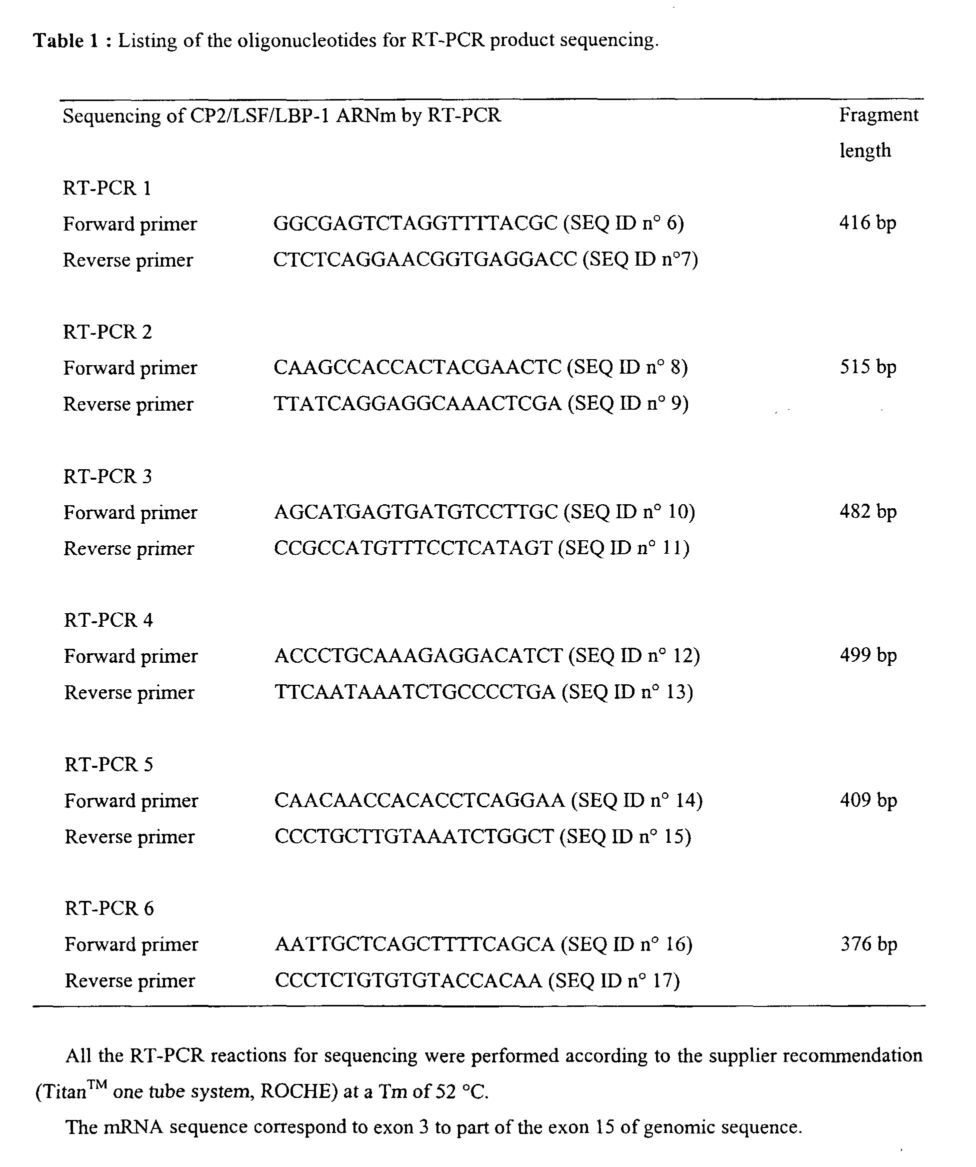

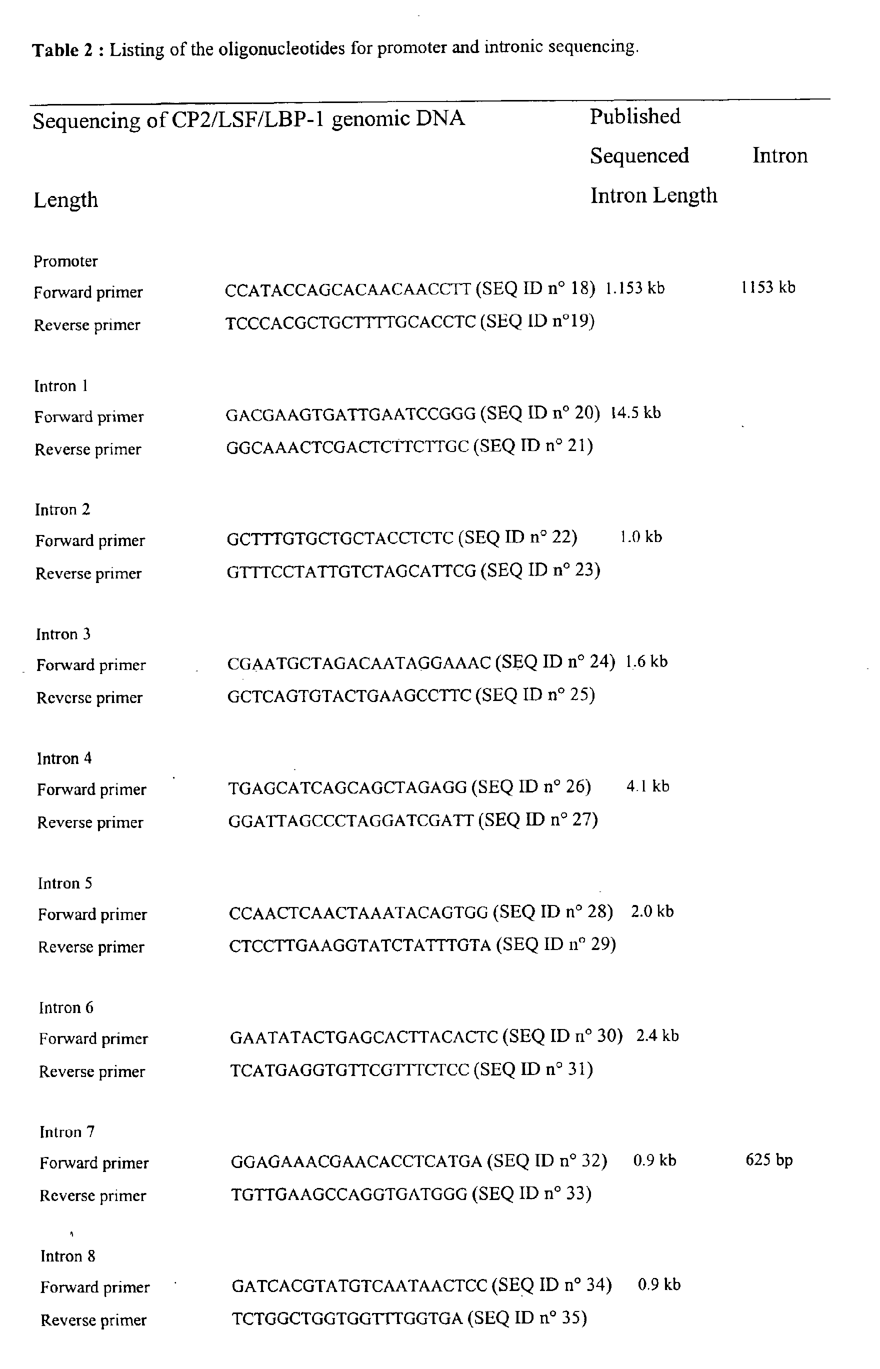

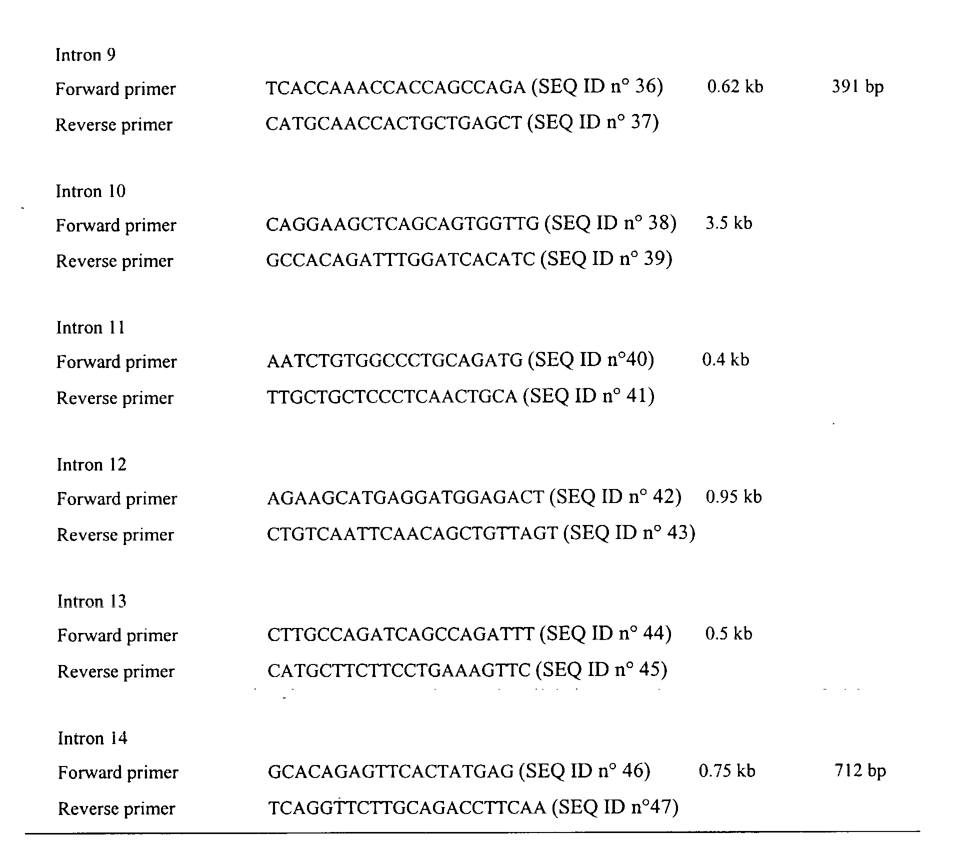

The nucleotidic sequence of intron 2 is SEQ ID n° 1.

The nucleotidic sequence of intron 3 is SEQ ID n° 2.

The nucleotidic sequence of intron 7 is SEQ ID n° 3.

The nucleotidic sequence of intron 9 is SEQ ID n° 4.

Numerous silent polymorphisms were discovered. The discovered polymorphisms are reported in Table 3.

**Table 3 :**

| Listing of the discovered polymorphisms. | | | | |
|---|---|---|---|---|
| | Polymorphism Frequency | Position | Characterisation | |
| | | | | |
| Promoter | G→A | -159^{a} | *Fok* I | 0.32 |
| | | | | |
| Exon 1 5'UTR | G→A | -354^{b} | - | 0.13 |
| | | | | |
| Intron 2 | A→G | +174^{c} | - | - |
| | G→C | +660^{c} | - | - |
| | | | | |
| Intron 3 | T→C | +210^{c} | *Pst* I | 0.33 |
| | T→G | +431^{c} | | |
| | | | | |
| Intron 7 | C→A | +134^{c} | *Fnu4H* I | - |
| | | | | |
| Intron 9 | A→G | -27^{d} | - | - |
| | | | | |
| Intron 10 | T→C | -14^{d} | - | - |
| | | | | |
| Exon 15 3'UTR | G→A | +15^{e} | *BspI286* I | 0.07 |

| | | | | |
|---|---|---|---|---|
| ^{a} from the putative TATA box described in Swendeman et al. | | | | |
| ^{b} from the first codon as described in Lim et al. | | | | |
| ^{c} from the donnor site. | | | | |
| ^{d} from the acceptor site. | | | | |
| ^{e} from the stop codon. Frequencies were established from a Caucasian control population and are reported in Table 4 | | | | |

**Table 4 :**

| | | Allele frequency | | | Genotype frequency | | | | | |
|---|---|---|---|---|---|---|---|---|---|---|
| ***APOE*** | n | ε2 | ε3 | ε4 | ε2/ε2 | ε2/ε3 | ε2/ε4 | ε3/ε3 | ε3/ε4 | ε4/ε4 |
| Control cases | 688 | 0.07 | 0.82 | 0.11 | 0.01 | 0.11 | 0.01 | 0.68 | 0.17 | 0.02 |
| AD cases | 709 | 0.04 | 0.62 | 0.34 | 0.00 | 0.03 | 0.04 | 0.40 | 0.41 | 0.12 |

| ***Th1/E47cs*** | n | G^{a} | T | | GG^{b} | GT | TT | | | |
|---|---|---|---|---|---|---|---|---|---|---|
| Control cases | 656 | 0.55 | 0.45 | | 0.31 | 0.47 | 0.22 | | | |
| AD cases | 657 | 0.44 | 0.56 | | 0.17 | 0.53 | 0.29 | | | |

| ***CP2pro*** | n | G^{c} | A | | GG^{d} | GA | AA | | | |
|---|---|---|---|---|---|---|---|---|---|---|
| Control cases | 642 | 0.67 | 0.13 | | 0.44 | 0.45 | 0.11 | | | |
| AD cases | 579 | 0.66 | 0.14 | | 0.44 | 0.44 | 0.12 | | | |

| ***CP2exon1*** | n | G | A | | GG | GA | AA | | | |
|---|---|---|---|---|---|---|---|---|---|---|
| Control cases | 664 | 0.87 | 0.13 | | 0.76 | 0.22 | 0.02 | | | |
| AD cases | 660 | 0.89 | 0.11 | | 0.79 | 0.19 | 0.02 | | | |

| ***CP2exon15*** | n | G | A | | GG | GA | AA | | | |
|---|---|---|---|---|---|---|---|---|---|---|
| Control cases | 680 | 0.94 | 0.06 | | 0.88 | 0.12 | 0.00 | | | |
| AD cases | 704 | 0.97 | 0.03 | | 0.94 | 0.06 | 0.00 | | | |

### Example 2:

### Implication of CP2 polymorphisms in Alzheimer's disease

We tested in a case-control study three of them located in the promoter (CP2pro), in the exon 1 of the 5'UTR (CP2exon1), in the exon 15 of the 3' untranslated terminal region (CP2exon15).

As already described the presence of the ε4 allele was an important risk factor (OR=5.23 95% Cl=4.18-6.77, p<0.0001), while the possession of the ε2 allele exhibited a protective effect (OR=0.49 95% Cl=0.34-0.71, *P*<0.0002). The presence of the Th1/E47cs T allele was strongly associated with Alzheimer's disease in this population (OR=2.11 95% Cl=1.63-2.73, *P*<0.0001), independently of the ε4 allele. The frequency of the CP2Pro A allele corresponds to 33% of the control group and no variation of allelic and genotypic distributions was observed between patients and controls. The frequency of the CP2Exon1 A allele is of 13% of the control group. No significant variation in the distribution of this polymorphism was observed in cases of Alzheimer's disease compared with controls.

For the CP2exon15 polymorphism, the frequency of the A allele was of 6% in the control group. The frequency of this allele significantly decreased in Alzheimer's disease cases compared with controls (*P*<0.0001). The protective effect for carriers of at least A allele was 0.48 (95% Cl=0.33-0.70 ; *P*=0.0002 ; adjusted on age and gender). This effect was stronger in women (OR=0.37 95% Cl=0.22-0.63 ; *P*=0.0002 ; adjusted on age and gender) compared to men (OR=0.64 95% Cl=0.36-1.15 ; *P*=0<0.13) (table 5).

### Example 3 :

### Binding analysis of cytoplasmic and nuclear proteins to exon 15 alleles

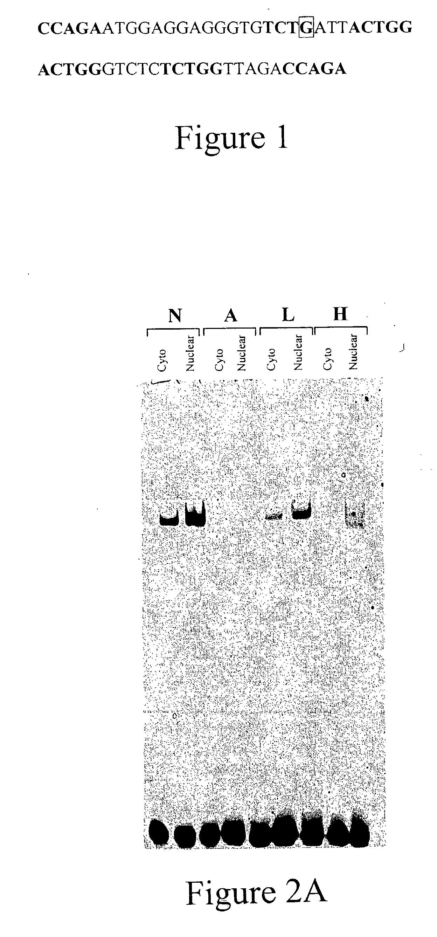

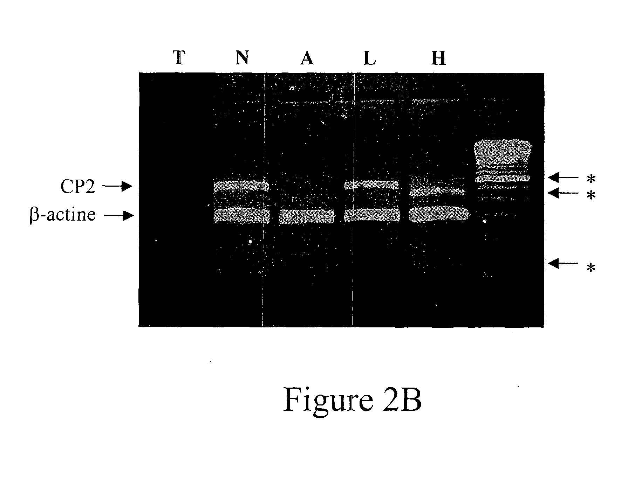

In order to investigate whether the exon15 GA allelic variation may be functional we performed the analyses of binding to cytoplasmic and nuclear proteins by electrophoretic mobility shift assays. We tested different cells lines (fig 2) : neurobastoma (Kelly), astrocytoma (85HG66), hepatoma (HEPG2) cells and lymphocytes from controls and cases of Alzheimer's disease. We observed a protein binding using both cytoplasmic and nuclear extracts from neuroblastoma and lymphocytes. Conversely, the protein binding was weak and not detectable using nuclear extracts from hepatoma and astrocytoma cells, respectively. The CP2 mRNA expression was analysed in these different cell lines and interestingly the presence of CP2 mRNA seems to correlate to the level of binding of the interacting protein (fig. 2b).

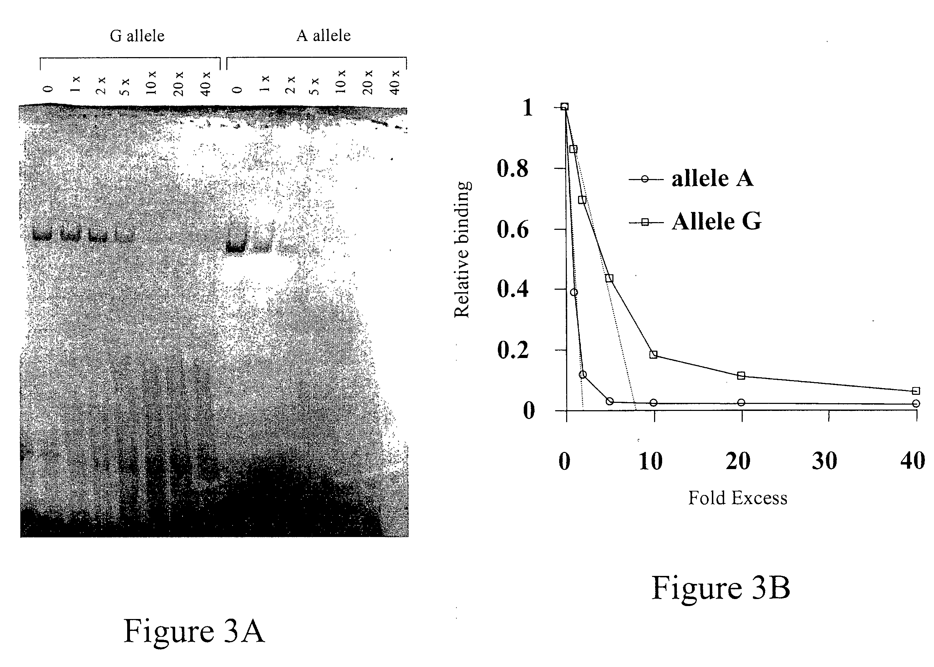

Using nuclear extract from neuroblastoma we tested whether this protein binding could be different according to exon 15 alleles at the equilibrium (Fig 3). We compared the A and G alleles with respect to their affinity for the nuclear proteins. The A allele displayed a 3.75 fold lower affinity (average of 3.5 and 4 fold) to the nuclear proteins than the G allele under equilibrium conditions (fig 3a,b).

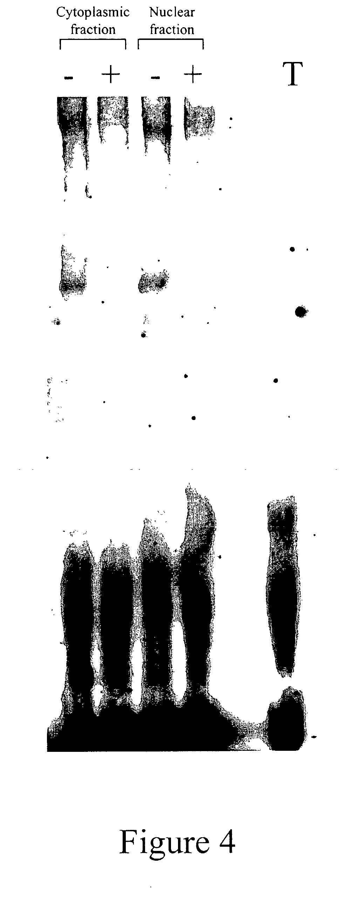

Since the Cp2 is a protein playing a role in the inflammatory process, we tested if the protein binding to the CP2exon15 polymorphic sequence differ after a treatment with Phyto-hemaglutinin (PHA) of lymphocytes from a random population. We observed a high decrease in this binding after such treatment, suggesting that the production of this protein is regulated by the inflammatory process (Figure 4).

### Example 4 :

### Level of expression of CP2 gene in lymphocyte from control and AD patients according to the CP2 exon 15 genotype.

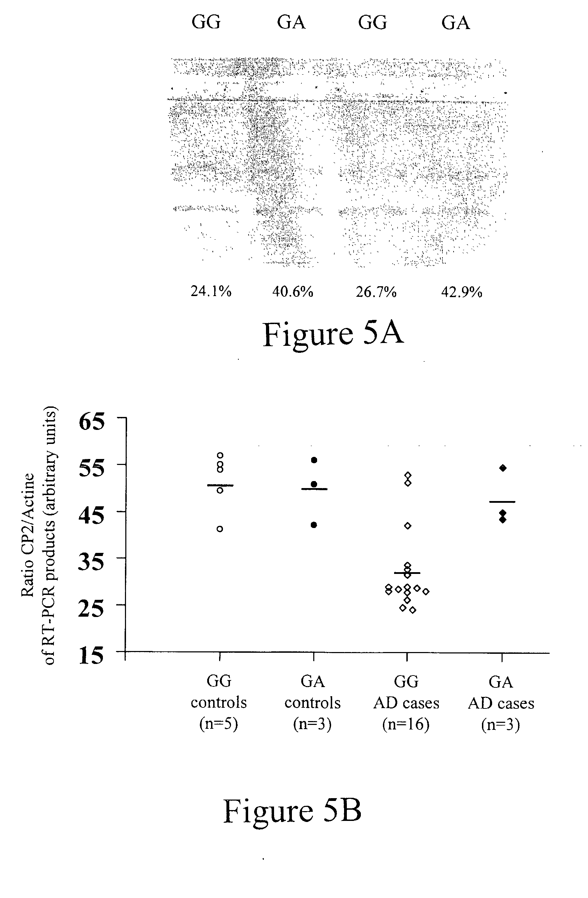

In order to better define the impact of the CP2exon15 polymorphism on the level of expression of the CP2 gene, we estimated by RT-PCR, the ratio between the amount of the CP2 and β-actin mRNA from lymphocytes of control and AD cases (Figure 5). The level of expression of the CP2 gene was decreased in AD cases compared to controls (50.6±6.1 % and 35.9±10.8 % respectively, *P*<0.005 with a non parametric Wilcoxon test). However, AD cases carrying the GA genotype did not exhibit this difference (47.7±6.1 %, *P*<0.9), conversely to those bearing the GG genotype (33.8±10.2 %, *P*<0.002 with a non parametric Wilcoxon test). So, a borderline significant decrease of this expression appeared in the AD cases according to the CP2exon15 genotype (GG=33.8±10.2 versus GA=47.7±6.1, P<0.06 with a non parametric Wilcoxon test).

These data suggested that a decrease of the CP2 expression may be associated with AD development while the protective effect of the A allele may result of a maintenance of the CP2 expression.

### Example 5 :

### Interaction between Th1/E47cs polymorphisms and CP2 polymorphism

Since the Th1/E47cs polymorphism is located in a consensus binding sequence of the CP2 factor, we tested potential interaction between both polymorphisms. We detected a borderline significant interaction (*P*<0.05). Therefore individuals carriers of at least an exon15 A allele and genotyped Th1/E47cs TT presented the weaker risk of developing Alzheimer's disease (OR=0.29 95%CI=0.13-0.64; *P*<0.002), while the carriers of the Th1/E47cs GG genotype did not exhibit a protective effect of the exon15 A allele (OR=0.88 95% Cl=0.41-1.11; *P*<0.7), the carriers of the Th1/E47cs GT genotype presenting intermediate impact of the exon 15 A allele (OR=0.48 95% Cl=0.28-0.83 ; *P*<0.009). Furthermore, as expected, no increased risk was associated with the Th1/E47cs T allele when individuals carried at least one exon 15 A allele (OR=1.03 95% Cl=0.44-2.42; *P*<0.9), confirming the protective effect of the exon 15 A allele.

### REFERENCES

Bing, Z., Reddy, S.A., Ren, Y., Qin, J. and Liao, W.S. Purification and characterization of the serum amyloid A3 enhancer factor. *J.Biol.Chem.* 274:24649-24656, 1999.

Bullido, M.J., Artiga, M.J., Recuero, M., et al. A polymorphism in the regulatory region of APOE associated with risk for Alzheimer's dementia. *Nat.Genet.* 18:69-71, 1998.

Chodosh, L.A., Baldwin, A.S., Carthew, R.W. and Sharp, P.A. Human CCAAT-binding proteins have heterologous subunits. *Cell* 53:11-24, 1988.

Cruts, M., van Duijn, C.M., Backhovens, H., et al. Estimation of the genetic contribution of presenilin-1 and -2 mutations in a population-based study of presenile Alzheimer disease. *Hum*.*Mol*.*Genet*. 7:43-51, 1998.

Farrer, L.A., Cupples, L.A., Haines, J.L., et al. Effects of age, sex, and ethnicity on the association between apolipoprotein E genotype and Alzheimer disease. A meta-analysis. APOE and Alzheimer Disease Meta Analysis Consortium [see comments]. *JAMA* 278:1349-1356, 1997.

Goate, A., Chartier-Harlin, M.C., Mullan, M., et al. Segregation of a missense mutation in the amyloid precursor protein gene with familial Alzheimer's disease [see comments]. *Nature* 349:704-706, 1991.

Huang, H.C., Sundseth, R. and Hansen, U. Transcription factor LSF binds two variant bipartite sites within the SV40 late promoter. *Genes Dev.* 4:287-298, 1990.

Kato, H., Horikoshi, M. and Roeder, R.G. Repression of HIV-1 transcription by a cellular protein. *Science* 251:1476-1479, 1991.

Lambert, J.C., Pasquier, F., Cottel, D., Frigard, B., Amouyel, P. and Chartier-Harlin, M.C. A new polymorphism in the APOE promoter associated with risk of developing Alzheimer's Disease. *Hum.Mol.Genet.* 7:533-540, 1998a.

Lambert, J.C., Wavrant-De, V.F., Amouyel, P. and Chartier-Harlin, M.C. Association at LRP gene locus with sporadic late-onset Alzheimer's disease. *Lancet* 351:1787-1788, 1998b.

Levy-Lahad, E., Wasco, W., Poorkaj, P., et al. Candidate gene for the chromosome 1 familial Alzheimer's disease locus [see comments]. *Science* 269:973-977, 1995.

Lim, L.C., Swendeman, S.L. and Sheffery, M. Molecular cloning of the alpha-globin transcription factor CP2. *Mol*.*Cell Biol.* 12:828-835, 1992.

Parada, C.A., Yoon, J.B. and Roeder, R.G. A novel LBP-1-mediated restriction of HIV-1 transcription at the level of elongation in vitro. *J*.*Biol*.*Chem*. 270:2274-2283, 1995.

Pericak-Vance, M.A., Bass, M.P., Yamaoka, L.H., et al. Complete genomic screen in late-onset familial Alzheimer disease. Evidence for a new locus on chromosome 12 [see comments]. *JAMA* 278:1237-1241, 1997.

Rogaeva, E., Premkumar, S., Song, Y., et al. Evidence for an Alzheimer disease susceptibility locus on chromosome 12 and for further locus heterogeneity [see comments]. *JAMA* 280:614-618, 1998.

Russo, T., Faraonio, R., Minopoli, G., De Candia, P., De Renzis, S. and Zambrano, N. Fe65 and the protein network centered around the cytosolic domain of the Alzheimer's beta-amyloid precursor protein. *FEBS Lett*. 434:1-7, 1998.

Saunders, A.M., Strittmatter, W.J., Schmechel, D., et al. Association of apolipoprotein E allele epsilon 4 with late-onset familial and sporadic Alzheimer's disease [see comments]. *Neurology* 43:1467-1472, 1993.

Sherrington, R., Rogaev, E.l., Liang, Y., et al. Cloning of a gene bearing missense mutations in early-onset familial Alzheimer's disease [see comments]. *Nature* 375:754-760, 1995.

Swendeman, S.L., Spielholz, C., Jenkins, N.A., Gilbert, D.J., Copeland, N.G. and Sheffery, M. Characterization of the genomic structure, chromosomal location, promoter, and development expression of the alpha-globin transcription factor CP2. *J*.*Biol*.*Chem*. 269:11663-11671, 1994.

Wavrant-DeVrieze, F., Lambert, J.C., Stas, L., et al. Association between coding variability in the LRP gene and the risk of late-onset Alzheimer's disease. *Hum. Genet.* 104:432-434, 1999.

Wu, W.S., Holmans, P., Wavrant-DeVrieze, F., et al. Genetic studies on chromosome 12 in late-onset Alzheimer disease [see comments]. *JAMA* 280:619-622, 1998.

### Annex to the application documents - subsequently filed sequences listing

acctccttga gatctttaga gaagcctcag tgtctttttt gatacttagt cttatagcaa 360
ggaaaacaga agctactttg atgaagagca gttctcttag tctagattaa cagttaggat 420
tttgataatt cacagcacag ttgaaacaga ggagttttgt caaaaggtgg tgggaggaat 480
taataggcac ttctacaaag taagagtaac aaaaggaaag ttgatggaaa caggctctac 540
ctaccctcan tgtttacagg ttgtatggtg ggttagcaag ccgggg 586

<210> 3
<211> 713
<212> DNA
<213> Homo sapiens

<220>
<221> intron
<222> (47)..(671)

<400> 3
gtggaganat acaggccttc cnatgaagac aaccatactc acagaggtaa aaagatttct 60
tttggtgaca atttcangtt cataattttt aatcttaaaa attcatcact tccaaactgg 120
tcagaattta cttctcctaa gccttgaggg acacagtatc acatggattc tgtgtccagc 180
ggccttaaca ggaagattgc tttagaattt ggcacgaacc atgccactgt ctctgtgtcc 240
aagttatctt tccccaaatt actctggttt ggtttggttt gctgcaggag ctattgtggt 300
gttctttgct ttgtatacat aagcacatct cttgcccaaa tagaattcag tttcatcttg 360
ggcataaacc accttcaatt ttaggaagag ctgtgtgctg cctttggttc tggaacccac 420
ttataaccaa caaaaatggc ctgggaccgt agttttctaa gacttatttc tttttagaag 480
tcctattctc aagcaggcct ccccacaggc tccaagatgg cggaaagaga agcagtgttc 540
tctattgctt atgggagact ctgaggaatt aagatttact aatcaagatg tgctattcaa 600
gtaacaatgt tttagtcaaa tcctggggtc atatgtgctc gttcattatc taacttatcc 660
tttgttttta gtgttctcca tggcccgaga tcacgtatgt ccgataactc aaa 713

<210> 4
<211> 451
<212> DNA
<213> Homo sapiens

<220>
<221> intron
<222> (19)..(349)

<400> 4
ncccccctcc gnccagatgt aagtctaaag ttgaatgtca gttacttgca atgtctggta 60
atactttgat gactgtccaa ttcagtagaa atgcccaact tttagggagt attattcatt 120
cattcattaa acaaacattt attgaatttt gactatgaac cagatactgt gctagggact 180
aaagatatga aacaagtaat gcaggacctt tccatttgag cagcttatag tctagtgggg 240
aagaaaagga tggaactcct tgagcatctt gcatggttgg agaaagagaa aacaactttc 300
acaaactagg cctgagtatg gggttgtgtg gggtgtttgg ctatgtgtta cctaatgggg 360
taacagacaa atctatttgg ttatctaaaa aacgtgctct ccctttcaga acctcttacc 420
aacaaccaca cctcaggaag ctcagcagtg g 451

<210> 5
<211> 438
<212> DNA
<213> Homo sapiens

<220>
<221> intron
<222> (1)..(368)

<211> 21
<212> DNA
<213> Homo sapiens

<400> 18
ccataccagc acaacaacct t                                                21

<210> 19
<211> 22
<212> DNA
<213> Homo sapiens

<400> 19
tcccacgctg cttttgcacc tc                                               22

<210> 20
<211> 21
<212> DNA
<213> Homo sapiens

<400> 20
gacgaagtga ttgaatccgg g                                                21

<210> 21
<211> 21
<212> DNA
<213> Homo sapiens

<400> 21
ggcaaactcg actcttcttg c                                                21

<210> 22
<211> 20
<212> DNA
<213> Homo sapiens

<400> 22
gctttgtgct gctacctctc                                                  20

<210> 23
<211> 22
<212> DNA
<213> Homo sapiens

<400> 23
gtttcctatt gtctagcatt cg                                               22

<210> 24
<211> 22
<212> DNA
<213> Homo sapiens

<400> 24
cgaatgctag acaataggaa ac                                               22

<400> 31
tcatgaggtg ttcgtttctc c                                                21

<210> 32
<211> 21
<212> DNA
<213> Homo sapiens

<400> 32
ggagaaacga acacctcatg a                                                21

<210> 33
<211> 20
<212> DNA
<213> Homo sapiens

<400> 33
tgttgaagcc aggtgatggg                                                  20

<210> 34
<211> 22
<212> DNA
<213> Homo sapiens

<400> 34
gatcacgtat gtcaataact cc                                               22

<210> 35
<211> 20
<212> DNA
<213> Homo sapiens

<400> 35
tctggctggt ggtttggtga                                                  20

<210> 36
<211> 20
<212> DNA
<213> Homo sapiens

<400> 36
tcaccaaacc accagccaga                                                  20

<210> 37
<211> 20
<212> DNA
<213> Homo sapiens

<400> 37
catgcaacca ctgctgagct                                                  20

<210> 38

<210> 45
<211> 21
<212> DNA
<213> Homo sapiens

<400> 45
catgcttctt cctgaaagtt c                                                21

<210> 46
<211> 19
<212> DNA
<213> Homo sapiens

<400> 46
gcacagagtt cactatgag                                                   19

<210> 47
<211> 22
<212> DNA
<213> Homo sapiens

<400> 47
tcaggttctt gcagaccttc aa                                               22

SEQUENCE LISTING

<110> Institut Pasteur de Lille
      INSERM

<120> Implication of a known gene named CP2/LSF-LBP-1 in
      Alzheimer's disease

<130> BET 99/1159

<140>
<141>

<160> 5

<170> PatentIn Ver. 2.1

<210> 1
<211> 910
<212> DNA
<213> Homo sapiens

<220>
<221> intron
<222> (35)..(910)

<400> 1
ctccatgatg aaaccctaac gtatctcaat caaggttaga tgcttcctca ttcattagta 60
ctattgcaga cctactgtat ttatgatttt tttgtttgtt tgtttgtttt gagacggcgt 120
cacccaggct ggagtgtagt ggcgccatct tggctcactg caagctctgc ctcccgggtt 180
cacaccattc tcctgcctca gcctcctaag tagctgggac cacaggcacc cgccaccaca 240
cccggctaat ttttttgtat ttttagtaga gatagggttt catcgtgtta gccaggatgg 300
tctcgatctc ctgacctcat gatccgcctg ccttggcctc ccaaagtgct gggattacag 360
gcgtgagcca ccgtgcccgg ccatttatga tgttttatta ggtacactta tatgtaaaaa 420
cttagatctt tctagtgtgg cctatcaaac tagaagtcct acttcagaga tttgagttct 480
aatgtcatac ttgatatcta ttactagctt cttttgctta gctgtccatt tttagtgtcc 540
agcttcctct tcctaatcat attccctaga cacttccatt ggttactggc tgagaagtat 600
tactctacct ggctctatta tttaccaatt gtgtggcctt ggacaaagtt gctaaaactc 660
ttttaagttt caatttcttt ggctgtaaaa tgggattggg attgataata ccttagaaaa 720
gtatatcagt taaatgagat aatgtgacaa tacttagcac agtgctgagc atcttgtaaa 780
ggcttctgat tgttgttact aatactaata atattattat actgttgtat attcaaccaa 840
ctgaatttta ttctttccga atctagcaaa gttaatttgt ggtgaatata atatgtaatt 900
acttttctt 910

<210> 2
<211> 586
<212> DNA
<213> Homo sapiens

<220>
<221> intron
<222> (13)..(486)

<400> 2
aaattggtga aggtaaaacg aataaatcat tcttctgtac aatatttttt actaatcctt 60
ttgggatttt aaattttgtt gttgttgtta atgttctttt gcttccaatg gcttaatggt 120
atctcttgcc tgcagtaatt tgttggcaga gtagtaagct gatgtgttat ttctttgggg 180
tcagagtaag ccccagtatt cacatgaatt tttcagagct gtagaaatag aacgcttacc 240
actccctgtc cagggtaatc acccctggct cagtgaagtt cttttgggtg gagtaaagta 300

acctccttga gatctttaga gaagcctcag tgtctttttt gatacttagt cttatagcaa 360
ggaaaacaga agctactttg atgaagagca gttctcttag tctagattaa cagttaggat 420
tttgataatt cacagcacag ttgaaacaga ggagttttgt caaaaggtgg tgggaggaat 480
taataggcac ttctacaaag taagagtaac aaaaggaaag ttgatggaaa caggctctac 540
ctaccctcan tgtttacagg ttgtatggtg ggttagcaag ccgggg 586

<210> 3
<211> 713
<212> DNA
<213> Homo sapiens

<220>
<221> intron
<222> (47)..(671)

<400> 3
gtggaganat acaggccttc cnatgaagac aaccatactc acagaggtaa aaagatttct 60
tttggtgaca atttcangtt cataattttt aatcttaaaa attcatcact tccaaactgg 120
tcagaattta cttctcctaa gccttgaggg acacagtatc acatggattc tgtgtccagc 180
ggccttaaca ggaagattgc tttagaattt ggcacgaacc atgccactgt ctctgtgtcc 240
aagttatctt tccccaaatt actctggttt ggtttggttt gctgcaggag ctattgtggt 300
gttctttgct ttgtatacat aagcacatct cttgcccaaa tagaattcag tttcatcttg 360
ggcataaacc accttcaatt ttaggaagag ctgtgtgctg cctttggttc tggaacccac 420
ttataaccaa caaaaatggc ctgggaccgt agttttctaa gacttatttc tttttagaag 480
tcctattctc aagcaggcct ccccacaggc tccaagatgg cggaaagaga agcagtgttc 540
tctattgctt atgggagact ctgaggaatt aagatttact aatcaagatg tgctattcaa 600
gtaacaatgt tttagtcaaa tcctggggtc atatgtgctc gttcattatc taacttatcc 660
tttgttttta gtgttctcca tggcccgaga tcacgtatgt ccgataactc aaa 713

<210> 4
<211> 451
<212> DNA
<213> Homo sapiens

<220>
<221> intron
<222> (19)..(349)

<400> 4
ncccccctcc gnccagatgt aagtctaaag ttgaatgtca gttacttgca atgtctggta 60
atactttgat gactgtccaa ttcagtagaa atgcccaact tttagggagt attattcatt 120
cattcattaa acaaacattt attgaatttt gactatgaac cagatactgt gctagggact 180
aaagatatga aacaagtaat gcaggacctt tccatttgag cagcttatag tctagtgggg 240
aagaaaagga tggaactcct tgagcatctt gcatggttgg agaaagagaa aacaactttc 300
acaaactagg cctgagtatg gggttgtgtg gggtgtttgg ctatgtgtta cctaatgggg 360
taacagacaa atctatttgg ttatctaaaa aacgtgctct ccctttcaga acctcttacc 420
aacaaccaca cctcaggaag ctcagcagtg g 451

<210> 5
<211> 438
<212> DNA
<213> Homo sapiens

<220>
<221> intron
<222> (1)..(368)

<400> 5
tgctcctggc ccaggatttt ttttttaaga gatggggtct tgctatgttg cccacgccta 60
acagtcttga actcctaaca gtcgattggc ctcaacaatc ctcctacctc agcttctcga 120
gtagctggga ctacaggcat gttgtgccca gcttagatag tggaaggtta tttcaggaca 180
gaaagaacaa cttgcattag acatcatcag tatgatgaaa gcatagggtt aagtggggag 240
gttaattggg gggaattgag acgggagagg agctaggagc caggtcatga agggccttat 300
atgccatgaa gaaaattatg gattgtaaca ttctggtaat gaggtgtcct tataatacag 360
cccattttaa tagtcattaa caaagagaat ctttgccttt atcatccatt ttcttatatt 420
tattccaggg gcagattt 438

SEQUENCE LISTING

<110> Institut Pasteur de Lille
      INSERM

<120> Implication of a known gene named CP2/LSF-LBP-1 in
      Alzheimer's disease

<130> BET 99/1159

<140>
<141>

<160> 47

<170> PatentIn Ver. 2.1

<210> 1
<211> 910
<212> DNA
<213> Homo sapiens

<220>
<221> intron
<222> (35)..(910)

<400> 1
   ctccatgatg aaaccctaac gtatctcaat caaggttaga tgcttcctca ttcattagta 60
   ctattgcaga cctactgtat ttatgatttt tttgtttgtt tgtttgtttt gagacggcgt 120
   cacccaggct ggagtgtagt ggcgccatct tggctcactg caagctctgc ctcccgggtt 180
   cacaccattc tcctgcctca gcctcctaag tagctgggac cacaggcacc cgccaccaca 240
   cccggctaat ttttttgtat ttttagtaga gatagggttt catcgtgtta gccaggatgg 300
   tctcgatctc ctgacctcat gatccgcctg ccttggcctc ccaaagtgct gggattacag 360
   gcgtgagcca ccgtgcccgg ccatttatga tgttttatta ggtacactta tatgtaaaaa 420
   cttagatctt tctagtgtgg cctatcaaac tagaagtcct acttcagaga tttgagttct 480
   aatgtcatac ttgatatcta ttactagctt cttttgctta gctgtccatt tttagtgtcc 540
   agcttcctct tcctaatcat attccctaga cacttccatt ggttactggc tgagaagtat 600
   tactctacct ggctctatta tttaccaatt gtgtggcctt ggacaaagtt gctaaaactc 660
   ttttaagttt caatttcttt ggctgtaaaa tgggattggg attgataata ccttagaaaa 720
   gtatatcagt taaatgagat aatgtgacaa tacttagcac agtgctgagc atcttgtaaa 780
   ggcttctgat tgttgttact aatactaata atattattat actgttgtat attcaaccaa 840
   ctgaatttta ttctttccga atctagcaaa gttaatttgt ggtgaatata atatgtaatt 900
   acttttcctt                                                         910

<210> 2
<211> 586
<212> DNA
<213> Homo sapiens

<220>
<221> intron
<222> (13)..(486)

<400> 2
aaattggtga aggtaaaacg aataaatcat tcttctgtac aatatttttt actaatcctt 60
ttgggatttt aaattttgtt gttgttgtta atgttctttt gcttccaatg gcttaatggt 120
atctcttgcc tgcagtaatt tgttggcaga gtagtaagct gatgtgttat ttctttgggg 180
tcagagtaag ccccagtatt cacatgaatt tttcagagct gtagaaatag aacgcttacc 240
actccctgtc cagggtaatc acccctggct cagtgaagtt cttttgggtg gagtaaagta 300

<400> 5
tgctcctggc ccaggatttt ttttttaaga gatggggtct tgctatgttg cccacgccta 60
acagtcttga actcctaaca gtcgattggc ctcaacaatc ctcctacctc agcttctcga 120
gtagctggga ctacaggcat gttgtgccca gcttagatag tggaaggtta tttcaggaca 180
gaaagaacaa cttgcattag acatcatcag tatgatgaaa gcatagggtt aagtggggag 240
gttaattggg gggaattgag acgggagagg agctaggagc caggtcatga agggccttat 300
atgccatgaa gaaaattatg gattgtaaca ttctggtaat gaggtgtcct tataatacag 360
cccattttaa tagtcattaa caaagagaat ctttgccttt atcatccatt ttcttatatt 420
tattccaggg gcagattt 438

<210> 6
<211> 20
<212> DNA
<213> Homo sapiens

<400> 6
ggcgagtcta ggttttacgc 20

<210> 7
<211> 21
<212> DNA
<213> Homo sapiens

<400> 7
ctctcaggaa cggtgaggac c 21

<210> 8
<211> 20
<212> DNA
<213> Homo sapiens

<400> 8
caagccacca ctacgaactc 20

<210> 9
<211> 20
<212> DNA
<213> Homo sapiens

<400> 9
ttatcaggag gcaaactcga 20

<210> 10
<211> 20
<212> DNA
<213> Homo sapiens

<400> 10
agcatgagtg atgtccttgc 20

<210> 11
<211> 20
<212> DNA
<213> Homo sapiens

<400> 11
ccgccatgtt tcctcatagt                                                    20

<210> 12
<211> 20
<212> DNA
<213> Homo sapiens

<400> 12
accctgcaaa gaggacatct                                                    20

<210> 13
<211> 20
<212> DNA
<213> Homo sapiens

<400> 13
ttcaataaat ctgcccctga                                                    20

<210> 14
<211> 20
<212> DNA
<213> Homo sapiens

<400> 14
caacaaccac acctcaggaa                                                    20

<210> 15
<211> 20
<212> DNA
<213> Homo sapiens

<400> 15
ccctgcttgt aaatctggct                                                    20

<210> 16
<211> 20
<212> DNA
<213> Homo sapiens

<400> 16
aattgctcag cttttcagca                                                    20

<210> 17
<211> 19
<212> DNA
<213> Homo sapiens

<400> 17
ccctctgtgt gtaccacaa                                                     19

<210> 18

<210> 25
<211> 21
<212> DNA
<213> Homo sapiens

<400> 25
gctcagtgta ctgaagcctt c                                                21


<210> 26
<211> 20
<212> DNA
<213> Homo sapiens

<400> 26
tgagcatcag cagctagagg                                                  20


<210> 27
<211> 21
<212> DNA
<213> Homo sapiens

<400> 27
ggattagccc taggatcgat t                                                21


<210> 28
<211> 22
<212> DNA
<213> Homo sapiens

<400> 28
ccaactcaac taaatacagt gg                                               22


<210> 29
<211> 23
<212> DNA
<213> Homo sapiens

<400> 29
ctccttgaag gtatctattt gta                                              23


<210> 30
<211> 23
<212> DNA
<213> Homo sapiens

<400> 30
gaatatactg agcacttaca ctc                                              23


<210> 31
<211> 21
<212> DNA
<213> Homo sapiens

<211> 21
<212> DNA
<213> Homo sapiens

<400> 38
caggaagctc agcagtggtt g                                                21

<210> 39
<211> 21
<212> DNA
<213> Homo sapiens

<400> 39
gccacagatt tggatcacat c                                                21

<210> 40
<211> 20
<212> DNA
<213> Homo sapiens

<400> 40
aatctgtggc cctgcagatg                                                  20

<210> 41
<211> 20
<212> DNA
<213> Homo sapiens

<400> 41
ttgctgctcc ctcaactgca                                                  20

<210> 42
<211> 21
<212> DNA
<213> Homo sapiens

<400> 42
agaagcatga ggatggagac t                                                21

<210> 43
<211> 23
<212> DNA
<213> Homo sapiens

<400> 43
ctgtcaattc aacagctgtt agt                                              23

<210> 44
<211> 21
<212> DNA
<213> Homo sapiens

<400> 44
cttgccagat cagccagatt t                                                21

## Claims

1. Method of predicting an increased risk of a human subject of developing Alzheimer's disease comprising assaying for a mutation within the ADN sequence of the CP2/LSF/LBP-1 gene including the region controlling the expression of said gene.

2. A method according to claim 1, wherein said mutation is a polymorphic form of said gene.

3. A method according to claim 1 or claim 2, wherein the mutation is present in a non coding sequence of the gene.

4. A method according to anyone of claims 1 to 3, wherein the mutation is present in an intron.

5. A method according to anyone of claims 1 to 3, wherein the mutation is present in the promoter region of the CP2/LSF-LBP-1 gene.

6. A method according to anyone of claims 1 to 3, wherein the mutation is present in the 5' untranslated region of exon 1.

7. A method according to anyone of claims 1 to 3, wherein the mutation is present in the 3' untranslated region of exon 15.

8. A method according to claim 7, wherein the mutation is in position +15 of the stop codon of exon 15 of the CP2/LFS-LBP-1 gene, namely a Go A polymorphism in said position.

9. A method according to claims 1 to 8, comprising the amplification of a region of interest suspected to comprise said mutation, by a polymerase chain reaction by means of oligonucleotide primers, wherein the reaction product comprises polynucleotides of at least 25 contigous nucleotides from said CP2/LSF-LBP-1 gene.

10. A method according to claim 9, further comprising sequencing gel reaction products form a PCR or RT-PCR reaction, namely the PCR *Bsp* 12861 digest from a PCR or RT-PCR reaction.

11. A method according to anyone of claims 1 to 10, further comprising one or more of the following steps :
- determining the genotype of the apolipoprotein E, the presence of at least one copy of the ε4 allele being indicative of an increased risk of developing Alzheimer's disease ;
- determining the genotype of the Th1/E47cs polymorphism, the presence of at least one copy of the G allele (T→G mutation in position -186 from the TATA box of the APOE gene) being indicative of an increased risk of developing Alzheimer's disease.

12. A method of screening for an agent capable of treating Alzheimer's disease comprising contacting a cultured cell line comprising a mutation or an allelic variant of the CP2/LSF-LBP-1 gene with an agent capable of treating Alzheimer's disease and monitoring the expression or processing of proteins encoded by the mutated form or allelic variant of the CP2/LSF-LBP-1 gene.

13. An isolated nucleic acid comprising at least 15, preferably at least 20 consecutive nucleotides of the cDNA of the LBP-1 gene having at least one of the following mutations or polymorphisms :
G (+ 637) → A (exon 1)
G (+ 2089) → A (exon 15)

14. An isolated nucleic acid selected from :
- nucleic acids comprising at least 15, preferably at least 20 consecutive nucleotides of the cDNA sequence of the promoter of the LBP-1 gene comprising the G (+ 120) → A polymorphism ;
- nucleic acids comprising at least 15, preferably at least 20 consecutive nucleotides of sequence SEQ ID n° 1 (intron 2) comprising one or more polymorphisms selected from A (+ 174) → G and G (+ 660) → C ;
- nucleic acids comprising at least 15, preferably at least 20 consecutive nucleotides of sequence SEQ ID n° 2 (intron 3) comprising one or more polymorphisms selected from T (+ 210) → C and T (+ 431) → G ;
- nucleic acids comprising at least 15, preferably at least 20 consecutive nucleotides of sequence SEQ ID n° 3 (intron 7) comprising the C (+ 134) → A polymorphism ;
- nucleic acids comprising at least 15, preferably at least 20 consecutive nucleotides of sequence SEQ ID n° 4 (intron 9) comprising the A (-27) → G polymorphism ;
- nucleic acids comprising at least 15, preferably at least 20 consecutive nucleotides of sequence SEQ ID n° 5 (intron 10) comprising the T (- 14) → pC polymorphism.

15. A vector comprising a nucleic acid according to claim 13 or 14.

16. A transgenic host comprising a nucleic acid according to claim 13 or 14.

17. A transgenic host according to claim 16 which is a primary or immortalized eukaryotic cell line.

18. A transgenic host according to claim 16 which is a bacterium.

19. A transgenic host according to claim 16 wherein the nucleic acid is integrated into the host genome.

20. A transgenic non human animal, preferably mammal with germlines or somatic cells comprising a heterologous gene encompassing a mutated CP2/LSF-LBP-1 gene as defined in claim 13 or 14, namely the CP2/LSF-LBP-1 exon 15mutant defined in claim 13.

21. A method for the identification of human subjects suffering from Alzheimer's disease to be responsive to a given therapy, namely cholinomimetic therapy comprising :
a) determining the genotype of the CP2/LSF-LBP-1 gene ; and optionally one or more of the following steps :
b) determining the genotype of the apolipoprotein E ;
c) determining the genotype of the Th1/E47cs polymorphism ; and
d) determining the phase of the apolipoprotein E and Th1/E47cs polymorphism.
